# EUROPEAN PATENT APPLICATION

(11) **EP 4 817 529 A1**
(43) Date of publication of application: **07.10.2026**
(21) Application number: 26165040.2
(22) Date of filing: 16.03.2026
(51) Int. Cl.: A61B 8/08, A61B 8/00

(54) **MEDICAL SUPPORT DEVICE, MEDICAL SUPPORT METHOD, AND MEDICAL SUPPORT PROGRAM**

(30) Priority: 31.03.2025 JP 2025059244
(71) Applicant: FUJIFILM Corporation, Tokyo 106-8620 (JP)
(72) Inventor: SASUGA, Saeko, Tokyo (JP)
(74) Representative: Dehns Germany Partnerschaft mbB

(57) **Abstract**

Provided are a medical support device, a medical support method, and a medical support program that can support an operation of an ultrasound probe.

A medical support device (11) includes: a processor, in which the processor is configured to acquire a surgical field image (21) captured by a camera (13B), the surgical field image (21) showing a state in which a surgical field is being scanned with an ultrasound probe (14), derive orientation information of the ultrasound probe (14) based on the surgical field image (21), and adjust a display mode of an ultrasound image (22) captured by the ultrasound probe (14) based on the orientation information.

## Description

### Technical Field

The present disclosure relates to a medical support device, a medical support method, and a medical support program.

### Background Art

In the related art, a technology of identifying a position and an orientation of a medical instrument and supporting medical care such as a surgery and an examination based on the identified position and orientation of the medical instrument is known.

For example, JP2024-131034A discloses deriving position and orientation information of a medical device within a captured image based on a marker included in the captured image, and displaying a composite image in which medical support information is superimposed at a position identified within the captured image based on the position and orientation information. In addition, for example, JP2025-003543A discloses subjecting at least one of a radiographic image or an ultrasound image to at least a rotation process such that a region of interest captured in the radiographic image and a region of interest captured in the ultrasound image have the same orientation as each other.

### SUMMARY

In general, in a case where an ultrasound probe is brought into contact with a body surface to perform imaging, a surgeon and a subject in a supine position often take a posture in which the surgeon and the subject face each other, that is, a posture in which the surgeon directs a line of sight from a tail side toward a head side of the subject. In this case, correspondence between upper and lower directions and left and right directions of an ultrasound image displayed on a display appears natural to the surgeon. Specifically, a right side of a body of the subject corresponds to a left side on the ultrasound image, and a left side of the body of the subject corresponds to a right side on the ultrasound image. This makes it possible to intuitively know a direction in which the ultrasound image moves in a case where the ultrasound probe is moved in the left-right direction, and the operation is facilitated.

In recent years, for example, as in a laparoscopic surgery and a robotic surgery, the ultrasound probe is operated via a surgical field image captured by a camera. In such a surgery, a position and an orientation of the ultrasound probe may be restricted, and a direction of the ultrasound probe cannot be determined in a fixed direction as viewed from the viewpoint of the surgeon, that is, the viewpoint of the camera. Therefore, a movement direction of the ultrasound probe on the surgical field image and a movement direction of the ultrasound image may deviate from each other, and the operation may be difficult to intuitively perform.

The present disclosure provides a medical support device, a medical support method, and a medical support program that can support an operation of an ultrasound probe.

According to a first aspect of the present disclosure, there is provided a medical support device comprising: a processor, wherein the processor is configured to acquire a surgical field image captured by a camera, the surgical field image showing a state in which a surgical field is being scanned with an ultrasound probe, derive orientation information of the ultrasound probe based on the surgical field image, and adjust a display mode of an ultrasound image captured by the ultrasound probe based on the orientation information.

The processor may be configured to calculate an angle between a direction of the ultrasound probe indicated by the orientation information and a predetermined reference direction, and adjust the display mode depending on whether or not the angle satisfies a predetermined criterion.

The processor may be configured to perform control of displaying the ultrasound image in a left-right reversed or up-down reversed manner in a case where the angle is equal to or greater than a predetermined threshold value.

The reference direction may be a predetermined direction in a three-dimensional coordinate system, and the processor may be configured to derive three-dimensional orientation information of the ultrasound probe based on the surgical field image.

The reference direction may be a predetermined direction in a two-dimensional coordinate system on the surgical field image, and the processor may be configured to derive two-dimensional orientation information of the ultrasound probe based on the surgical field image.

The processor may be configured to perform control of displaying information indicating a direction of the ultrasound probe around the ultrasound image or displaying and superimposing the information on the ultrasound image, based on the orientation information.

The processor may be configured to determine over time whether or not the ultrasound probe is in contact with a target part to be imaged by the ultrasound probe, adjust the display mode in a separated state in which the ultrasound probe is separated from the target part, and fix the display mode in a contact state in which the ultrasound probe is in contact with the target part.

The processor may be configured to determine whether the ultrasound probe is in the separated state or in the contact state based on at least one of an electric signal corresponding to a reflected wave of an ultrasound wave transmitted to the target part by the ultrasound probe or the ultrasound image.

The processor may be configured to derive the orientation information and position information of the ultrasound probe in the surgical field based on the surgical field image, and perform control of displaying a superimposed image obtained by superimposing the ultrasound image on the surgical field image, the superimposed image including the ultrasound image whose display mode and display position are adjusted based on the orientation information and the position information.

The camera may be an endoscope, and the ultrasound probe may be inserted into a body and image an internal structure of a target part in the body while in contact with the target part.

According to a second aspect of the present disclosure, there is provided a medical support method executed by a computer, the medical support method comprising: acquiring a surgical field image captured by a camera, the surgical field image showing a state in which a surgical field is being scanned with an ultrasound probe; deriving orientation information of the ultrasound probe based on the surgical field image; and adjusting a display mode of an ultrasound image captured by the ultrasound probe based on the orientation information.

According to a third aspect of the present disclosure, there is provided a medical support program for causing a computer to execute: acquiring a surgical field image captured by a camera, the surgical field image showing a state in which a surgical field is being scanned with an ultrasound probe; deriving orientation information of the ultrasound probe based on the surgical field image; and adjusting a display mode of an ultrasound image captured by the ultrasound probe based on the orientation information.

According to the above aspects, the medical support device, the medical support method, and the medical support program of the present disclosure can support the operation of the ultrasound probe.

### BRIEF DESCRIPTION OF DRAWINGS

FIG. 1 is a diagram showing a state of an inside of a body in a robotic surgery.
FIG. 2 is a diagram showing an example of a hardware configuration of a medical support device.
FIG. 3 is a diagram showing an example of a functional configuration of the medical support device.
FIG. 4 is a diagram showing a relationship in a position and an orientation between a marker and an ultrasound probe.
FIG. 5 is a diagram showing an example of a surgical field image.
FIG. 6 is a diagram showing a relationship in a position and an orientation between a marker and an ultrasound probe.
FIG. 7 is a diagram showing an example of a surgical field image.
FIG. 8 is a diagram for describing a correspondence relationship between a movement direction of the ultrasound probe and a movement direction of an ultrasound image.
FIG. 9 is a diagram for describing a correspondence relationship between a movement direction of the ultrasound probe and a movement direction of an ultrasound image.
FIG. 10 is a diagram for describing a correspondence relationship between a movement direction of the ultrasound probe and a movement direction of an ultrasound image that is left-right reversed.
FIG. 11 is a diagram showing an example of a screen displayed on a display.
FIG. 12 is a diagram for describing processing of a determination unit.
FIG. 13 is a flowchart showing an example of medical support processing.
FIG. 14 is a diagram showing an example of a screen displayed on the display in a second embodiment.
FIG. 15 is a diagram showing an example of a screen displayed on the display in the second embodiment.
FIG. 16 is a diagram showing a modification example of a screen displayed on the display in the second embodiment.
FIG. 17 is a diagram showing an example of a screen displayed on the display in a third embodiment.
FIG. 18 is a diagram showing an example of a screen displayed on the display in the third embodiment.

### DESCRIPTION OF EMBODIMENTS

Hereinafter, an example of an embodiment of the disclosed technology will be described with reference to the drawings. The same or equivalent components and parts in the respective drawings are denoted by the same reference numerals, and the duplicated description will be omitted. In addition, dimensional ratios in the drawings are exaggerated for convenience of description and may be different from the actual ratios.

### First Embodiment

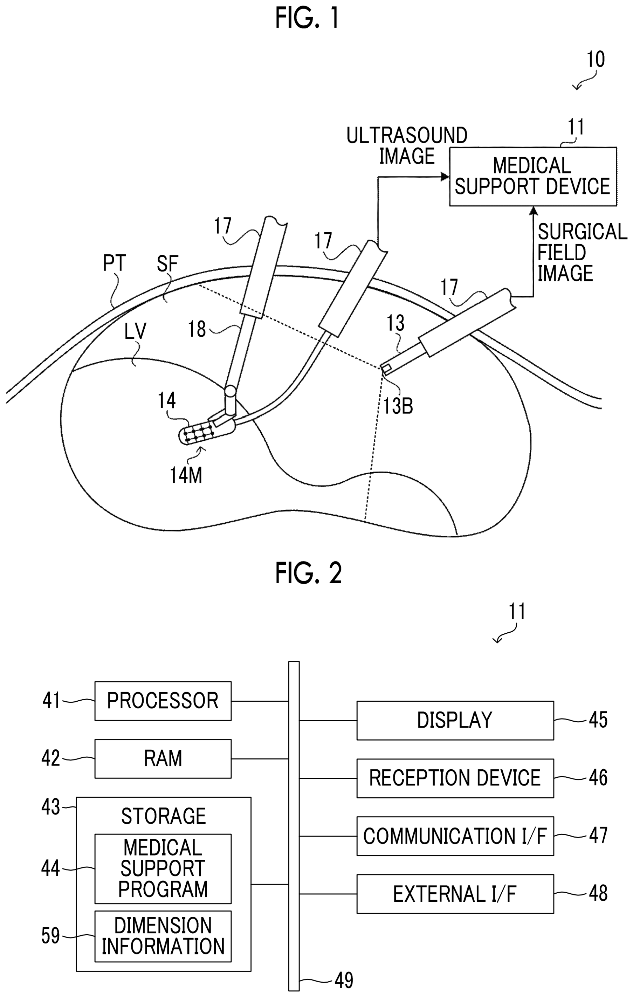

An example of a medical support system 10 to which a medical support device 11 according to the present embodiment is applied will be described with reference to FIG. 1. The medical support system comprises the medical support device 11, an endoscope 13, and an ultrasound probe 14. The medical support device 11 is communicably connected to the endoscope 13 and the ultrasound probe 14.

As an example, the medical support system is used in a case where a surgery using an endoscopic surgery support robot (hereinafter, referred to as a robotic surgery) is performed on a patient PT. The robotic surgery involves creating a plurality of small holes in a body of the patient PT, inserting various medical instruments connected to robotic arms through the holes, and having a surgeon remotely control instruments via a dedicated console or the like.

The medical support system 10 provides a medical staff including a surgeon with a field of view of a surgical field SF inside the body of the patient PT, and with support information for supporting medical care such as a surgery and an examination. The support information is, as described below, an ultrasound image 22 corresponding to a direction of the ultrasound probe 14 on a surgical field image 21, and the like. Such a medical support system 10 has a function of providing the support information in real time during a surgery, and is therefore also called a surgical navigation system or the like.

FIG. 1 shows a state in which the endoscope 13, the ultrasound probe 14, and forceps 18 are inserted into the abdomen of the patient PT. These various medical instruments are inserted into the body via a trocar 17. In addition, pneumoperitoneum is performed by injecting carbon dioxide into the abdominal cavity. The trocar 17 is an insertion tool having an insertion hole into which various medical instruments are inserted and a valve provided in the insertion hole to prevent gas leakage.

In the example of FIG. 1, the forceps 18 grip the ultrasound probe 14. A position and an orientation of the ultrasound probe 14 are controlled by operating a robot arm (not shown) connected to the forceps 18. The medical instrument used in the medical support system 10 is not limited to the endoscope 13, the ultrasound probe 14, and the forceps 18. For example, a biopsy needle, a tissue sampling needle, an injection needle, a snare, an electric scalpel, or a high-frequency knife may be applied.

The endoscope 13 optically images a surgical field SF including a target part (in this example, a liver LV) inside the body of the patient PT by using a camera. The surgical field SF is a space that spreads in a body cavity defined by an organ and a body wall inside the body. Specifically, the endoscope 13 has an insertion part to be inserted into the body of the patient PT. A camera 13B and a light source (for example, a light emitting diode (LED)) for illumination are incorporated in a distal end part of the insertion part. The endoscope 13 is, for example, a rigid endoscope in which the insertion part is rigid, and is often used for abdominal cavity observation, so that the endoscope 13 is also called a laparoscope.

The camera 13B has an image sensor such as a charge coupled device (CCD) image sensor and a complementary metal oxide semiconductor (CMOS) image sensor, and an imaging optical system including a lens that forms a subject image on an imaging surface of the image sensor. The image sensor is, for example, an image sensor capable of capturing a color image. The endoscope 13 is connected to an image processing processor for an endoscope (not shown). This image processing processor performs signal processing on an imaging signal output by the image sensor to generate a surgical field image 21 of the surgical field SF inside the body. The surgical field image 21 captured by the endoscope 13 is transmitted to the medical support device 11 in real time via the image processing processor for an endoscope. The camera 13B of the endoscope 13 is an example of a "camera" of the present disclosure.

As illumination light for the endoscope 13, for example, visible light such as white light is used. As the illumination light of the endoscope 13, special light such as ultraviolet light and infrared light may be used. As the special light, for example, light restricted to a specific wavelength such as short-wavelength narrow-band light obtained by narrowing down light in a short wavelength range such as an ultraviolet range may be used. The surgical field image 21 is a video of the surgical field SF illuminated with illumination light, and more specifically, is a video based on light reflected from the illumination light near the surface of the surgical field SF. Therefore, in the surgical field image 21, a structure present in the vicinity of a surface layer of the target part can be visualized, but it is difficult to observe an internal structure.

The ultrasound probe 14 transmits an ultrasound wave to the target part while in contact with the target part in the body and detects an electric signal corresponding to an ultrasound echo reflected from the target part, thereby imaging an internal structure of the target part. Specifically, the ultrasound probe 14 has an ultrasound transducer. The ultrasound transducer transmits an ultrasound wave to the target part and receives an ultrasound echo reflected from the target part. The ultrasound probe 14 is connected to an image processing processor for an ultrasound probe (not shown). This image processing processor performs image reconstruction processing based on an electric signal corresponding to the ultrasound echo received by the ultrasound transducer so that an ultrasound image corresponding to the electric signal is generated. Through the image reconstruction processing, the ultrasound image 22 showing an internal structure of the target part scanned by the ultrasound probe 14 is generated. The ultrasound image 22 captured by the ultrasound probe 14 is transmitted to the medical support device 11 in real time via the image processing processor for an ultrasound probe.

The ultrasound probe 14 is, for example, a convex type that radially transmits ultrasound waves, and acquires a fan-shaped ultrasound image 22 with the ultrasound transducer as a base point. A plurality of the ultrasound images 22 are captured along a scanning direction by performing the scanning with the ultrasound probe 14. The ultrasound image 22 is a so-called brightness (B)-mode image in which an internal structure of the target part, from a surface layer to a deep layer where the ultrasound wave reaches, is visualized as brightness information. The ultrasound image 22 visualizes an internal structure of the target part that cannot be observed in the surgical field image 21 obtained by optical imaging.

The medical support device 11 acquires the surgical field image 21 from the endoscope 13, and acquires the ultrasound image 22 from the ultrasound probe 14. The medical staff is provided with the visual field inside the body of the patient PT through the screen of the display. In the robotic surgery, the surgeon performs a surgery, an examination, and the like by operating the robot arm via the dedicated console while referring to these images.

Hereinafter, a detailed configuration of the medical support device 11 will be described. FIG. 2 shows an example of a hardware configuration of the medical support device 11. The medical support device 11 comprises a processor 41, a random access memory (RAM) 42, a storage 43, a display 45, a reception device 46, a communication interface (I/F) 47, and an external I/F 48. These units are connected to a bus 49 such as a system bus and a control bus, and can communicate with each other.

The processor 41 is, for example, a central processing unit (CPU), and integrally controls the respective units of the medical support device 11 in accordance with a control program and executes various types of processing in accordance with various types of application programs. The processor 41 is an example of a "processor" of the present disclosure.

The RAM 42 is a memory that transitorily stores information, and is used as a work memory by the processor 41. Examples of the RAM 42 include a dynamic random access memory (DRAM) and a static random access memory (SRAM).

The storage 43 is a non-volatile storage device that stores various programs, various parameters, and the like. Examples of the storage 43 include a hard disk drive (HDD) and a solid state drive (SSD). The storage 43 stores a medical support program 44 for causing a computer to function as the medical support device 11.

In addition, the storage 43 stores dimension information 59. The dimension information 59 includes information about a geometric size and shape of a medical instrument (the ultrasound probe 14, the forceps 18, and the like) to which a marker described below is assigned. This is, for example, information indicating a length, a width, a diameter, a height, a thickness, and a structural feature (linear or curved shape) of the entire medical instrument and/or a specific part (such as distal end part) of the medical instrument. In addition, the dimension information 59 includes information about a shape of the marker and placement of the marker on the medical instrument. This is, for example, information indicating how the marker is provided in an axial direction and a circumferential direction of the medical instrument.

Various types of information including the surgical field image 21 and the ultrasound image 22 are displayed on the display 45. Examples of the display 45 include a liquid crystal display and an electro-luminescence (EL) display. The number of the displays 45 is not particularly limited, and may be one or more.

The reception device 46 includes a keyboard, a mouse, and the like (not shown), and receives an instruction from an operator. That is, the medical support device 11 is operated by an operator such as the medical staff through the reception device 46. The reception device 46 may be a device that receives touch input, such as a touch panel, a device that receives voice input, such as a microphone, a device that receives gesture input, such as a camera, or the like.

The communication I/F 47 is connected to a network (not shown) such as a local area network (LAN) and/or a wide area network (WAN), and performs transmission control in accordance with a communication protocol defined in various types of wired or wireless communication standards. The external I/F 48 is, for example, a universal serial bus (USB) interface, and is used for connection to peripheral devices such as a printer and a memory card. As the medical support device 11, for example, a personal computer, a server computer, a smartphone, a tablet terminal, or a wearable terminal can be applied as appropriate.

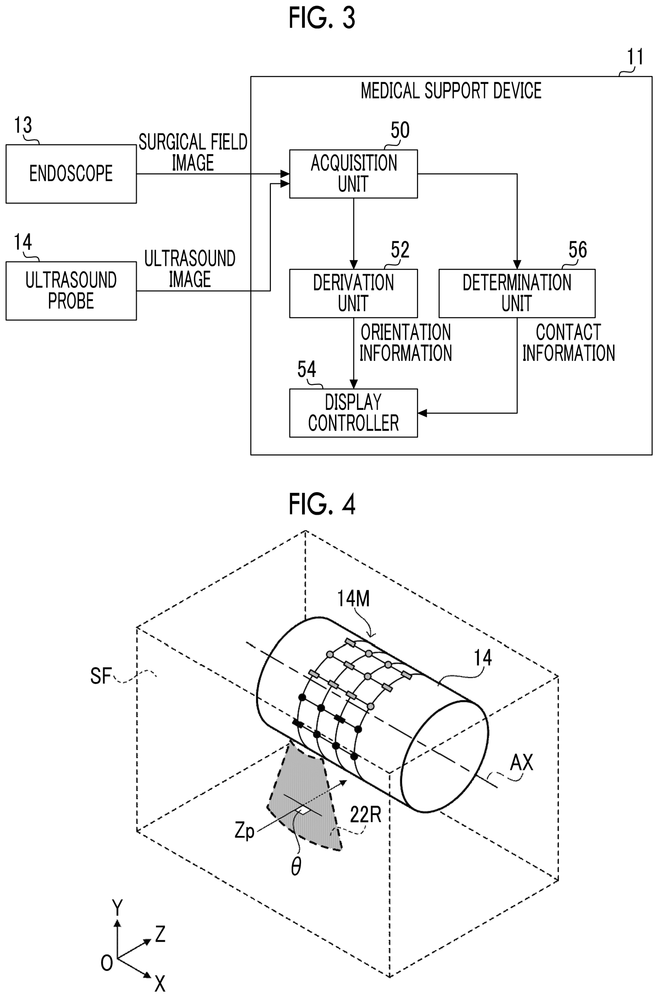

Next, a functional configuration of the medical support device 11 will be described. FIG. 3 is a block diagram showing an example of the functional configuration of the medical support device 11. The medical support device 11 comprises an acquisition unit 50, a derivation unit 52, a display controller 54, and a determination unit 56 as functional units. The processor 41 reads out the medical support program 44 from the storage 43 and executes the medical support program 44 on the RAM 42, so that the processor 41 functions as each functional unit. The processor 41 operates as each functional unit to implement the medical support processing.

The acquisition unit 50 acquires a surgical field image 21 captured by the camera 13B, the surgical field image 21 showing a state in which the surgical field SF is being scanned with the ultrasound probe 14. For example, the acquisition unit 50 acquires the surgical field image 21 from a device including a processor of the endoscope 13 via the external I/F 48 or the communication I/F 47. The processor of the endoscope 13 may be provided in the medical support device 11.

In addition, the acquisition unit 50 acquires the ultrasound image 22 captured by the ultrasound probe 14. For example, the acquisition unit 50 acquires the ultrasound image 22 from a device including a processor of the ultrasound probe 14 via the external I/F 48 or the communication I/F 47. The processor of the ultrasound probe 14 may be provided in the medical support device 11.

The derivation unit 52 derives orientation information of the ultrasound probe 14 based on the surgical field image 21. Specifically, the derivation unit 52 derives three-dimensional orientation information of the ultrasound probe 14 based on the surgical field image 21. Further, the derivation unit 52 may derive position information of the ultrasound probe 14 in the surgical field SF based on the surgical field image 21.

For example, the derivation unit 52 derives the orientation information and the position information of the ultrasound probe 14 based on a marker 14M included in the surgical field image 21. The marker 14M is a marker that is recognizable from the surgical field image 21 optically captured by the camera 13B and is assigned to the ultrasound probe 14. In addition, information about a shape of the marker 14M and placement of the marker 14M on the ultrasound probe 14 is known, and is stored in advance in the storage 43 as the dimension information 59.

Specifically, the derivation unit 52 detects the marker 14M by searching for a morphological feature of the marker 14M from the surgical field image 21. For example, the derivation unit 52 may detect the marker 14M by using an image processing method such as pattern matching. In addition, for example, the derivation unit 52 may detect the marker 14M by using an artificial intelligence (AI) technology using a machine learning model. As such a machine learning model, for example, a neural network model such as a convolutional neural network (CNN) that is trained in advance to receive the surgical field image 21 as an input and output a region of the marker 14M in the input surgical field image 21 can be applied.

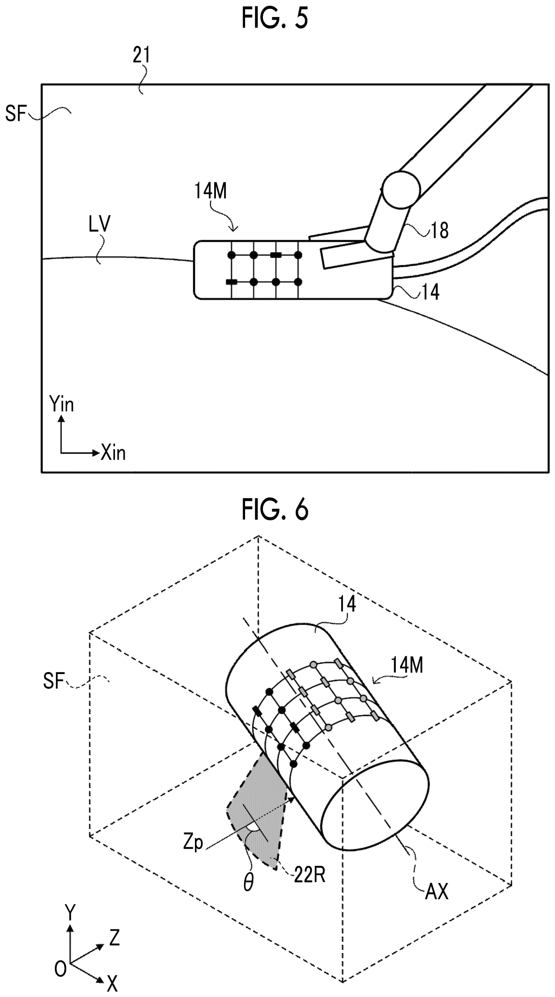

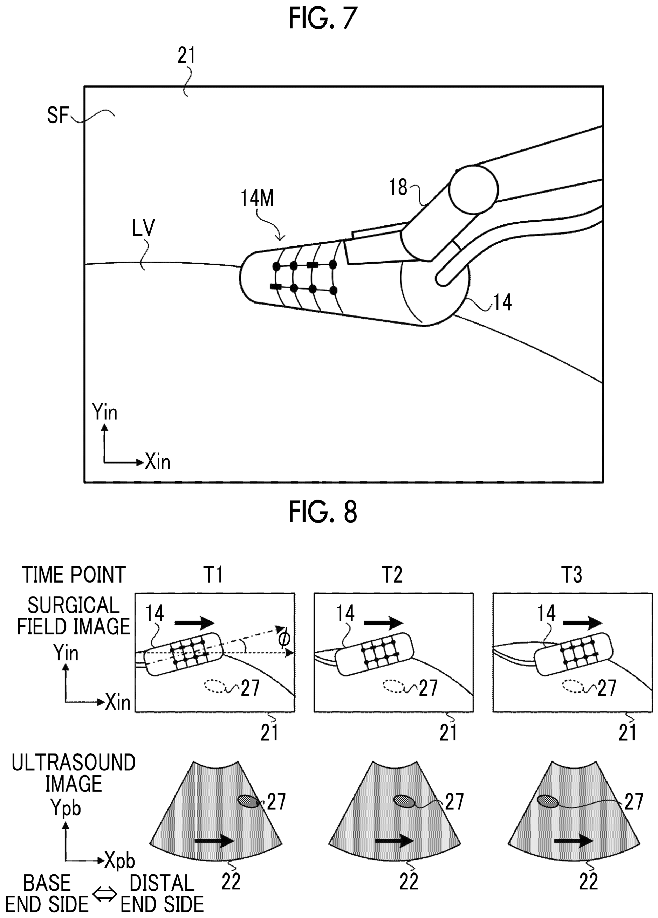

Then, the derivation unit 52 derives the orientation information and the position information of the ultrasound probe 14 based on the detected marker 14M. A method of deriving the orientation information and the position information will be described with reference to FIGS. 4 to 7. FIGS. 4 and 6 are diagrams conceptually showing a distal end part of the ultrasound probe 14 in the surgical field SF defined as a three-dimensional space, each of which assumes a different position and orientation. FIG. 5 shows a surgical field image 21 captured in a case where the position and the orientation of the distal end part of the ultrasound probe 14 are in the state of FIG. 4. FIG. 7 shows a surgical field image 21 captured in a case where the position and the orientation of the distal end part of the ultrasound probe 14 are in the state of FIG. 6.

As an example, the marker 14M is a marker of a lattice pattern composed of a first line extending in a direction of an axis AX of the distal end part of the ultrasound probe 14 and a second line orthogonal to the direction of the axis AX of the distal end part and formed in the circumferential direction along an outer peripheral surface of the distal end part. A circular symbol or a rectangular symbol is assigned to each intersection in the lattice pattern.

The marker 14M may include at least one of a line forming the lattice pattern or a symbol disposed at each intersection of the lattice pattern. In addition, the symbol on the marker 14M can have any shape, and may be represented by figures such as a triangle, a polygon, a star shape, and various marks, or may be represented by a character or the like. In addition, as the marker 14M, a two-dimensional code and a geometric pattern (for example, ArUco and ChArUco) may be used.

In the surgical field SF of the three-dimensional space shown in FIGS. 4 and 6, a Z-axis is a direction parallel to an imaging optical axis of the camera 13B. In addition, an X-Y plane in the surgical field SF is a plane parallel to an imaging plane (Xin-Yin plane) of the camera 13B and is orthogonal to the imaging optical axis. That is, the surgical field image 21 corresponds to a projection image obtained by projecting the surgical field SF from one viewpoint O. In FIGS. 4 and 6, among the symbols constituting the marker 14M, a symbol reflected in the surgical field image 21 is shown in a dark color, and a symbol not reflected in the surgical field image 21 is shown in a light color.

The orientation of the ultrasound probe 14 is detected, for example, as a direction of the axis AX of the distal end part of the ultrasound probe 14 in the surgical field SF. The direction of the axis AX is represented by, for example, an inclined angle with respect to each of an X-axis, a Y-axis, and a Z-axis.

For example, FIG. 4 shows a state in which the axis AX of the distal end part of the ultrasound probe 14 is parallel to the X-axis in the surgical field SF of the three-dimensional space. In this case, as shown in FIG. 5, the marker 14M shown in the surgical field image 21 has orthogonal lines of the lattice pattern parallel to the X-axis and Y-axis. In addition, the symbols forming the lattice pattern are shown in the surgical field image 21 at equal intervals.

On the other hand, FIG. 6 shows a state in which the axis AX of the distal end part of the ultrasound probe 14 is inclined with respect to the depth direction parallel to the imaging optical axis in the surgical field SF of the three-dimensional space. In this case, as shown in FIG. 7, in the marker 14M shown in the surgical field image 21, the farther the marker 14M is from the camera 13B in the depth direction, the shorter the line extending in the circumferential direction of the lattice pattern is, and the shorter the interval between the symbols forming the lattice pattern is.

As described above, the form of the marker 14M shown in the surgical field image 21 changes depending on the orientation of the ultrasound probe 14. The derivation unit 52 derives the direction of the axis AX of the distal end part of the ultrasound probe 14 as the orientation information based on the form (projected lattice pattern or the like) of the marker 14M that changes in this way in the surgical field image 21.

In addition, in a case where the position of the ultrasound probe 14 is changed in the surgical field SF, the position of the marker 14M shown in the surgical field image 21 is also changed. The derivation unit 52 may derive the position information of the ultrasound probe 14 in the surgical field SF based on the position of the marker 14M. The position of the ultrasound probe 14 is detected, for example, as position coordinates (X,Y,Z) of a predetermined reference point (for example, a distal end position) in the ultrasound probe 14.

An imaging distance (that is, a coordinate in the Z axis direction) from the camera 13B to the marker 14M can be calculated based on a focal length of the camera 13B and a size of the marker 14M shown in the surgical field image 21. Specifically, by associating the size of the marker 14M detected from the surgical field image 21 with the information on the marker 14M included in the dimension information 59, the amount of change in the projection scale can be estimated, and the imaging distance can be calculated. For example, the apparent size of the marker 14M is smaller as the distal end part is farther from the camera 13B and is larger as the distal end part is closer to the camera 13B. The derivation unit 52 derives the imaging distance by using such a geometric relationship.

FIGS. 4 and 6 also show an imaging range 22R of the ultrasound image 22 in the surgical field SF and an angle θ representing the direction of the ultrasound image 22. The angle θ is at its minimum in a case where the imaging optical axis (Z-axis) of the camera 13B is parallel to a display surface (Xpb-Ypb plane) of the ultrasound image 22, and is at its maximum in a case where the imaging optical axis is a normal line. In FIGS. 4 and 6, for ease of understanding, a straight line Zp parallel to the Z-axis passing through the display surface of the ultrasound image 22 is shown. The angle θ can be derived based on the orientation information of the ultrasound probe 14 and the dimension information 59.

A correspondence relationship between upper and lower directions and left and right directions of the ultrasound image 22 and the orientation of the ultrasound probe 14 is predetermined. Specifically, a horizontal direction (Xpb direction) of the ultrasound image 22 is associated with a longitudinal direction or a lateral direction of the ultrasound probe 14 as a reference. For example, it is assumed that a distal end side of the ultrasound probe 14 in the longitudinal direction is associated with a right side of the ultrasound image 22 and a base end side of the ultrasound probe 14 in the longitudinal direction is associated with a left side of the ultrasound image 22. In this case, in a case where the ultrasound probe 14 is moved left and right in an orientation in which the longitudinal direction of the ultrasound probe 14 is reflected in the surgical field image 21, the ultrasound image 22 also moves left and right.

Here, as shown in FIG. 8, a case is considered in which the surgeon moves the ultrasound probe 14 in the right direction (Xin direction, indicated by a thick arrow) on the surgical field image 21 while referring to the surgical field image 21 from a time point T1 to a time point T3. As in the example of FIG. 8, in an orientation in which the distal end side of the ultrasound probe 14 is positioned on a right side of the surgical field image 21, the imaging range of the ultrasound image 22 also moves in the right direction (Xpb direction, indicated by a thick arrow), so that the operation is easily performed intuitively. In FIG. 8, for ease of understanding, a position of a tumor 27 included in the ultrasound image 22 is also shown on the surgical field image 21 by a dotted line. In addition, the forceps 18 are not shown.

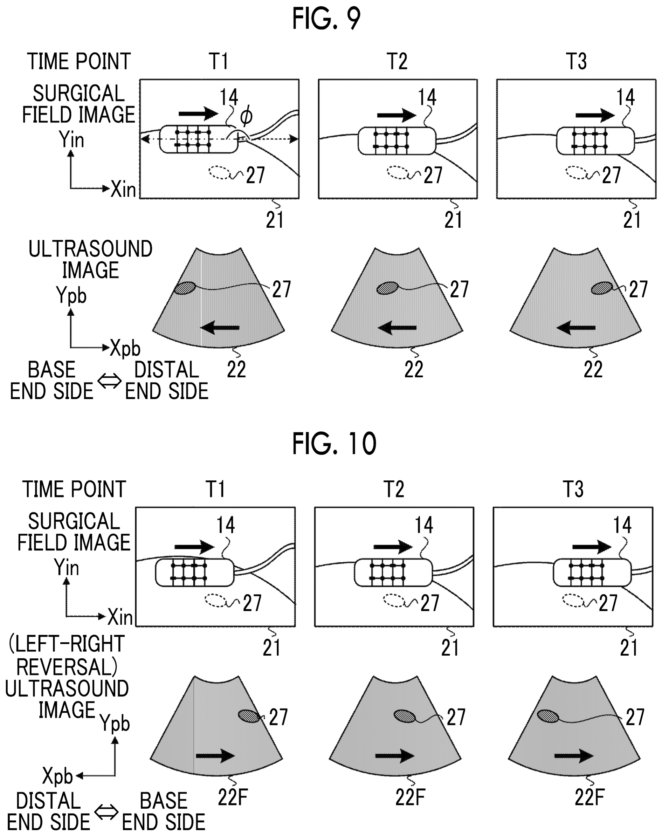

On the other hand, FIG. 9 shows an example in which the orientation of the ultrasound probe 14 is different from that of FIG. 8. As described above, a case is considered in which the surgeon moves the ultrasound probe 14 in the right direction (Xin direction, indicated by a thick arrow) on the surgical field image 21 from a time point T1 to a time point T3. As in the example of FIG. 9, in an orientation in which the distal end side of the ultrasound probe 14 is positioned on a left side of the surgical field image 21, the imaging range of the ultrasound image 22 moves in the left direction (negative Xpb direction, indicated by a thick arrow). That is, the movement direction of the ultrasound probe 14 and the movement of the ultrasound image 22 are opposite to each other, making intuitive operation difficult.

Therefore, the display controller 54 adjusts the display mode of the ultrasound image 22 captured by the ultrasound probe 14 based on the orientation information of the ultrasound probe 14. The phrase "adjusts the display mode" includes, for example, changing an appearance of the ultrasound image 22 itself, such as inverting, rotating, enlarging, reducing, and changing a display position on a screen of the ultrasound image 22. In addition, for example, it also includes assigning information indicating the direction of the ultrasound probe 14 to the periphery of the ultrasound image 22 while maintaining the ultrasound image 22 itself. As a result, the operation can be performed intuitively regardless of the orientation of the ultrasound probe 14.

Specifically, the display controller 54 calculates an angle φ between the orientation of the ultrasound probe 14 indicated by the orientation information and a predetermined reference direction. The orientation of the ultrasound probe 14 is represented by, for example, a vector extending from the base end side to the distal end side on the axis AX of the distal end part of the ultrasound probe 14. The reference direction is a predetermined direction in a three-dimensional coordinate system. In the examples of FIGS. 8 and 9, in a three-dimensional coordinate system (XYZ coordinate system) with the viewpoint O of the camera 13B as an origin, the angle φ is shown with a direction of the X axis of the surgical field SF, that is, a direction of the Xin axis on the surgical field image 21 (direction from the left side to the right side of the paper) as the reference direction. In FIG. 8, the angle φ is about 15 degrees, and, in FIG. 9, the angle φ is about 180 degrees.

Then, the display controller 54 adjusts the display mode of the ultrasound image 22 depending on whether or not the angle φ satisfies a predetermined criterion. For example, the display controller 54 may perform control of displaying the ultrasound image 22 in a left-right reversed manner in a case where the angle φ is equal to or greater than a predetermined threshold value. The threshold value can be optionally set, and, for example, in a case where the threshold value is set to 90 degrees, the display mode can be finely adjusted according to the orientation of the ultrasound probe 14. In addition, for example, in a case where the threshold value is set to about 91 to 120 degrees, the ultrasound image 22 can be reversed left and right only in a case where the orientation of the ultrasound probe 14 is significantly reversed.

FIG. 10 shows a state in which the ultrasound image 22 in the example of FIG. 9 is reversed left and right. In this case, the distal end side of the ultrasound probe 14 in the longitudinal direction is associated with a left side of an ultrasound image 22F after the left-right reversal, and the base end side of the ultrasound probe 14 in the longitudinal direction is associated with a right side of the ultrasound image 22F after the left-right reversal. As a result, in a case where the surgeon moves the ultrasound probe 14 in the right direction (Xin direction, indicated by a thick arrow) on the surgical field image 21 from a time point T1 to a time point T3, the movement direction of the ultrasound probe 14 matches the movement of the ultrasound image 22F after the left-right reversal. Therefore, an intuitive operation is possible.

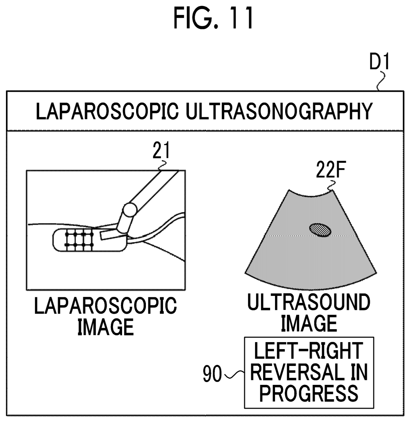

In addition, it is preferable that the display controller 54 notify of the fact that the ultrasound image 22 is reversed left and right. FIG. 11 shows an example of a screen D1 displayed on the display 45 by the display controller 54. The screen D1 includes the surgical field image 21, the ultrasound image 22F after the left-right reversal, and a notification 90 saying "left-right reversal in progress". As described above, by allowing the surgeon to immediately know whether the ultrasound image 22 is reversed left and right or not, more appropriate operation can be performed.

In addition, the display controller 54 may adjust the display mode of the ultrasound image 22 in stages according to the angle φ. For example, the display controller 54 may perform control of displaying the ultrasound image 22 as it is in a case where the angle φ is 0 to 90 degrees, displaying a warning and then displaying the ultrasound image 22 as it is in a case where the angle φ is 91 to 100 degrees, and displaying the ultrasound image 22F after the left-right reversal in a case where the angle φ is 101 degrees or more.

In addition, with respect to the angle φ, the display controller 54 may individually set a threshold value for starting the left-right reversal of the ultrasound image 22 and a threshold value for releasing the left-right reversal. In this case, it is preferable that the threshold value for starting be greater than the threshold value for releasing. By providing hysteresis in this way, in a case where the angle φ fluctuates around the threshold value, it is possible to prevent the display mode from being changed frequently.

In addition, the display controller 54 may refrain from the left-right reversal in a case where the angle φ is rapidly changing, that is, in a case where a time derivative of the angle φ is large, and may perform the left-right reversal of the ultrasound image 22 in a case where the time derivative of the angle φ is equal to or less than a predetermined threshold value. As a result, it is possible to prevent the display mode from being frequently changed in response to a temporary movement such as a camera shake.

In addition, in some cases, it is effective to perform up-down reversal depending on the insertion direction of the ultrasound probe 14 into the surgical field SF and the positional relationship with the camera 13B. For the up-down reversal, the display mode can be adjusted by the same process as for the left-right reversal. That is, the display controller 54 may perform control of displaying the ultrasound image 22 in an up-down reversed manner in a case where the angle φ is equal to or greater than a predetermined threshold value.

The correspondence relationship between the upper and lower directions of the ultrasound image 22 and the orientation of the ultrasound probe 14 described above is an example, and any setting can be made according to the usage form, the preference of the surgeon, and the like. For example, it may be set such that the distal end side of the ultrasound probe 14 in the longitudinal direction is associated with the left side of the ultrasound image 22 and the base end side of the ultrasound probe 14 in the longitudinal direction is associated with the right side of the ultrasound image 22. In addition, for example, it may be set such that the right side of the lateral direction as viewed from the base end side of the ultrasound probe 14 is associated with the right side of the ultrasound image 22 and the left side of the lateral direction as viewed from the base end side of the ultrasound probe 14 is associated with the left side of the ultrasound image 22.

In addition, the reference direction described above is an example, and any setting can be made according to the usage form, the preference of the operator, and the like. Specifically, the three-dimensional coordinate system for expressing the position and the orientation of the camera 13B and the ultrasound probe 14 can be optionally set, and for example, may be set according to at least one of the position or the orientation of the camera 13B or the ultrasound probe 14, or may be set as a world coordinate system. The reference direction is defined as a predetermined direction vector in the three-dimensional coordinate system, and is compared with the orientation of the ultrasound probe 14 expressed in the three-dimensional coordinate system.

Specifically, the three-dimensional coordinate system may be a local coordinate system (for example, a coordinate system in which the distal end part of the ultrasound probe 14 is an origin, the longitudinal direction is an X axis, the lateral direction is a Y axis, and the irradiation direction is a Z axis) set according to the position and the orientation of the ultrasound probe 14. In this case, the angle φ between the orientation of the ultrasound probe 14 and the reference direction can be calculated by converting the reference direction defined in the three-dimensional coordinate system (camera coordinate system) with the viewpoint O of the camera 13B as the origin as described above into the local coordinate system and projecting the reference direction. In addition, even in a case where the reference direction is a fixed direction (for example, a gravity direction and a horizontal direction) in the world coordinate system, the angle φ between the orientation of the ultrasound probe 14 and the reference direction can be calculated by converting the reference direction into the camera coordinate and/or the local coordinate system and projecting the reference direction.

### Modification Example

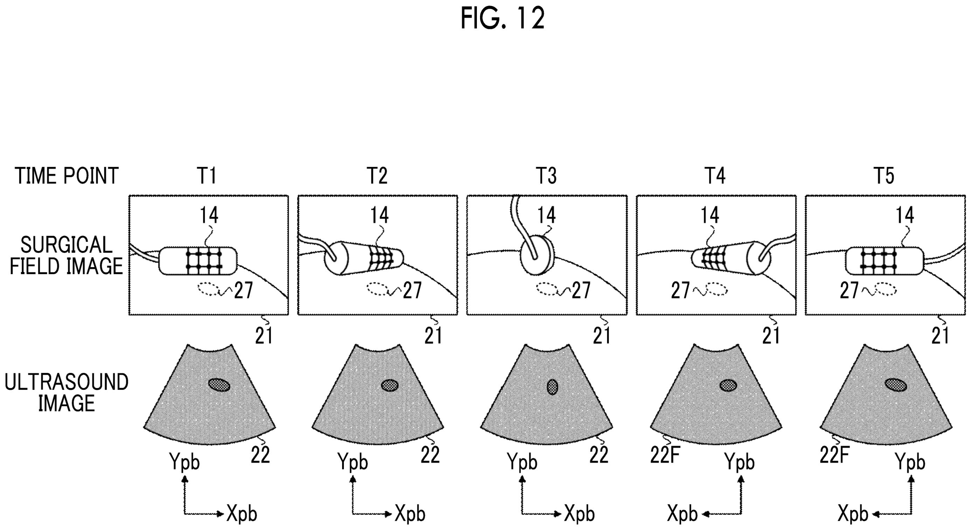

As shown in FIG. 12, imaging may be performed while changing the orientation of the ultrasound probe 14 with respect to one tumor 27 from a time point T1 to a time point T5. In such a scene in which images are continuously captured, in a case where the ultrasound image 22 is reversed left and right between a time point T3 and a time point T4, the visibility may be reduced. In particular, in a case where the direction of the axis AX of the distal end part of the ultrasound probe 14 and the imaging optical axis of the camera 13B are nearly parallel, as at a time point T3, the angle φ may fluctuate around the threshold value, causing the left-right reversal control to switch frequently, which may make it difficult for the surgeon to understand. Therefore, the medical support device 11 may be configured not to adjust the display mode of the ultrasound image 22 during the imaging by the ultrasound probe 14.

Specifically, the determination unit 56 may determine over time whether or not the ultrasound probe 14 is in contact with the target part to be imaged by the ultrasound probe 14. That is, the determination unit 56 may sequentially determine whether the ultrasound probe 14 is in a separated state in which the ultrasound probe 14 is separated from the target part or in a contact state in which the ultrasound probe 14 is in contact with the target part. The "contact" in the present disclosure is not limited to a state in which the ultrasound probe 14 and the target part are in close contact with each other, and may have a gap between the ultrasound probe 14 and the target part to a degree generally allowed in the technical field to which the technology of the present disclosure belongs.

A specific method of determining whether the ultrasound probe 14 is in the contact state or in the separated state is not particularly limited. Specific examples of the determination method will be described below, but the present disclosure is not limited to this, and a known method can be appropriately applied as the method of determining whether the ultrasound probe 14 is in the contact state or in the separated state. The determination unit 56 may combine some or all of the following determination methods as appropriate.

As an example, the determination unit 56 may determine whether the ultrasound probe 14 is in the separated state or in the contact state based on at least one of an electric signal corresponding to a reflected wave of an ultrasound wave transmitted to the target part by the ultrasound probe 14 or the ultrasound image 22. For example, the determination unit 56 may monitor frequency components of the detected electric signal over time, and may determine that the contact state and the separated state have been switched in a case where there is a change in the frequency components. In addition, for example, the frequency components that can be detected in the contact state may be stored in advance, and, in a case where the frequency components are included in the detected electrical signal, the determination unit 56 may determine that the state is the contact state.

In addition, for example, the determination unit 56 may monitor numerical information such as an average brightness value of the ultrasound image 22 over time, and may determine that the contact state and the separated state have been switched in a case where there is a change in the numerical information. In addition, for example, the determination unit 56 may detect the structure from the ultrasound image 22, and may determine that the contact state and the separated state have been switched in a case where there is a temporal change (for example, appearance or disappearance) in the shape of the structure. In addition, for example, the numerical information and/or the shape of the structure that can be observed in the contact state may be stored in advance, and, in a case where the numerical information and/or the structure is included in the ultrasound image 22, the determination unit 56 may determine that the state is the contact state.

As another example, the determination unit 56 may determine whether the ultrasound probe 14 is in the contact state or in the separated state by using a sensor such as a contact sensor or a proximity sensor. For example, by providing a sensor at a portion of the ultrasound probe 14 that can come into contact with the target part, the sensor can determine whether the ultrasound probe 14 is in the contact state or in the separated state. Examples of such a sensor include a photosensor, a contact sensor using a piezoelectric element, a capacitive proximity sensor, and a microswitch.

As another example, the determination unit 56 may determine whether the ultrasound probe 14 is in the contact state or in the separated state through image analysis based on the surgical field image 21 or the like. For example, the determination unit 56 may derive a distance between the ultrasound probe 14 and the target part from the surgical field image 21, determine that the ultrasound probe 14 is in the contact state in a case where the distance is less than a predetermined threshold value, and determine that the ultrasound probe 14 is in the separated state in a case where the distance is equal to or greater than the predetermined threshold value. In addition, for example, the determination as to whether the ultrasound probe 14 is in the contact state or in the separated state may be performed from the surgical field image 21 by using an AI technology using a machine learning model. As such a machine learning model, for example, a neural network model such as a CNN, which has been trained in advance to receive the surgical field image 21 as an input and output a determination result of the contact state or the separated state in the input surgical field image 21, can be applied. In addition, for example, a camera other than the endoscope 13 may be inserted into the body, and an image captured by the other camera may be analyzed in addition to or instead of the surgical field image 21 to determine whether the ultrasound probe 14 is in the contact state or in the separated state.

The display controller 54 may adjust the display mode of the ultrasound image 22 in the separated state in which the ultrasound probe 14 is separated from the target part. In other words, the display controller 54 may fix the display mode of the ultrasound image 22 in the contact state in which the ultrasound probe 14 is in contact with the target part, and may update the display mode only in a case where the ultrasound probe 14 enters the separated state. For example, in FIG. 12, the display controller 54 may continuously apply the display mode (without left-right reversal) determined at a time point T1 to a time point T2 to a time point T5.

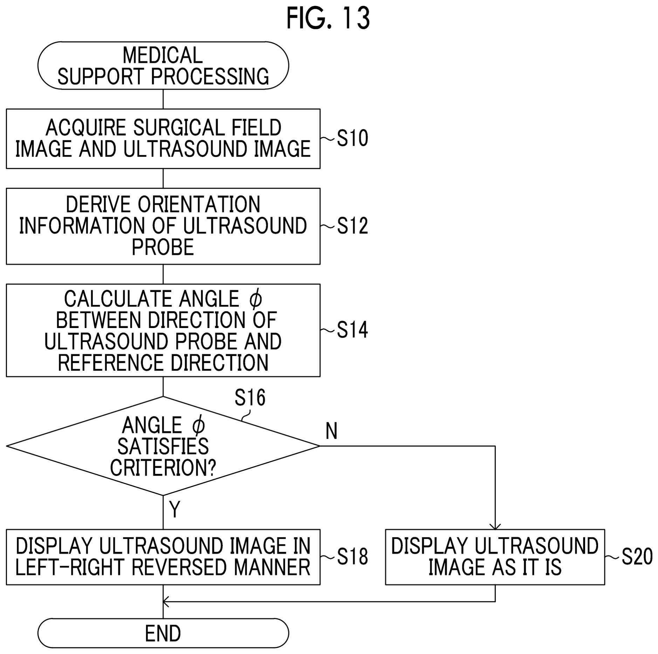

Next, an operation of the medical support device 11 according to the present embodiment will be described with reference to FIG. 13. In the medical support device 11, the medical support processing shown in FIG. 13 is executed by the processor 41 executing the medical support program 44. This processing is executed, for example, in a case where a user gives an instruction to start execution via the reception device 46.

In step S10, the acquisition unit 50 acquires the surgical field image 21 and the ultrasound image 22. In step S12, the derivation unit 52 derives the orientation information of the ultrasound probe 14 based on the surgical field image 21. In step S14, the display controller 54 calculates the angle φ between the orientation of the ultrasound probe 14 indicated by the orientation information and the predetermined reference direction.

In step S16, the display controller 54 determines whether or not the angle φ satisfies the predetermined criterion. In a case where the determination in step S16 is affirmative, the processing proceeds to step S18, the display controller 54 performs control of displaying the ultrasound image 22 in a left-right reversed (or up-down reversed) manner, and then ends this processing. In a case where the determination in step S16 is negative, the processing proceeds to step S20, the display controller 54 performs control of displaying the ultrasound image 22 as it is (without being reversed left and right and up and down), and then ends this processing.

As described above, the medical support device 11 according to the present embodiment comprises a processor, and the processor acquires a surgical field image 21 captured by the camera 13B, the surgical field image 21 showing a state in which the surgical field SF is being scanned with the ultrasound probe 14. The processor derives the orientation information of the ultrasound probe 14 based on the surgical field image 21. The processor adjusts the display mode of the ultrasound image 22 captured by the ultrasound probe 14 based on the orientation information.

With the medical support device 11 according to the present embodiment, the movement direction of the ultrasound probe 14 on the surgical field image 21 and the movement direction of the ultrasound image 22 can be associated with each other. That is, the correspondence relationship between the position inside the organ being observed in the ultrasound image 22 and the position of the ultrasound probe 14 in the surgical field SF can be simply and intuitively expressed, thereby improving the operability of the ultrasound probe 14 via the camera 13B. Therefore, it is possible to support the operation of the ultrasound probe 14.

### Second Embodiment

In the present embodiment, as another adjustment method of the display mode of the ultrasound image 22, a form in which information indicating the orientation of the ultrasound probe 14 is displayed will be described. In the following description, a part of the description overlapping with the first embodiment will be omitted.

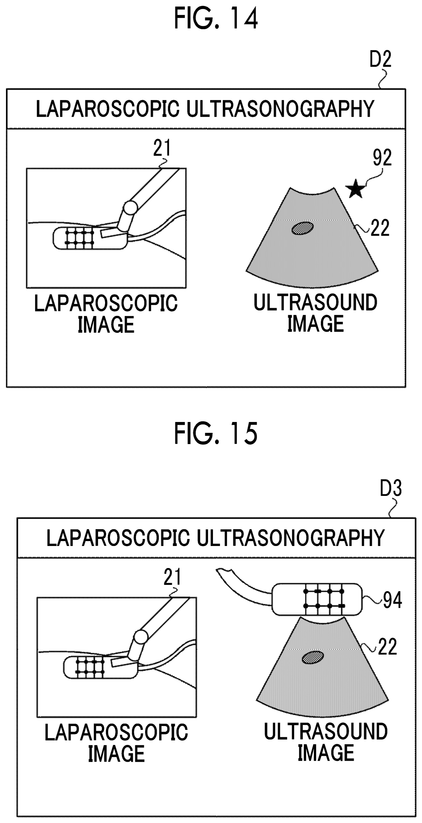

The display controller 54 performs control of displaying the information indicating the orientation of the ultrasound probe 14 around the ultrasound image 22 based on the orientation information of the ultrasound probe 14. FIGS. 14 and 15 show examples of a screen D2 and a screen D3 displayed on the display 45 by the display controller 54.

The screen D2 includes the surgical field image 21, the ultrasound image 22 that is neither reversed left and right nor reversed up and down, and an orientation mark 92. The orientation mark 92 is an example of the information indicating the orientation of the ultrasound probe 14, and is composed of a symbol, a figure, and the like indicating the distal end side (or the base end side) of the ultrasound probe 14. In the example of FIG. 14, the orientation mark 92 is a star-shaped figure meaning the distal end side of the ultrasound probe 14 in the longitudinal direction. In addition to or instead of the orientation mark 92, upper, lower, left, and right directions in the surgical field image 21 may be indicated by characters.

The screen D3 includes the surgical field image 21, the ultrasound image 22 that is neither reversed left and right nor reversed up and down, and a schematic diagram 94 of the ultrasound probe 14. The schematic diagram 94 is an example of the information indicating the orientation of the ultrasound probe 14, and is a diagram for intuitively indicating the orientation of the ultrasound probe 14.

With the orientation mark 92 and/or the schematic diagram 94, it is visually recognizable which direction on the screen the distal end side of the ultrasound probe 14 is pointing. As a result, for example, it is easy to understand that the ultrasound image 22 is moving toward the base end side of the ultrasound probe 14, even in a case where the ultrasound image 22 moves in the left direction in a case where the ultrasound probe 14 is moved in the right direction on the surgical field image 21. That is, the correspondence relationship between the position (for example, the tumor 27) inside the organ being observed in the ultrasound image 22 and the position of the ultrasound probe 14 in the surgical field SF can be simply and intuitively expressed, thereby improving the operability of the ultrasound probe 14 via the camera 13B. Therefore, it is possible to support the operation of the ultrasound probe 14.

The display controller 54 may perform control of superimposing and displaying the information indicating the orientation of the ultrasound probe 14 on the ultrasound image 22 based on the orientation information of the ultrasound probe 14. For example, in the screen D2 of FIG. 14, the orientation mark 92 may be displayed to be superimposed on the ultrasound image 22.

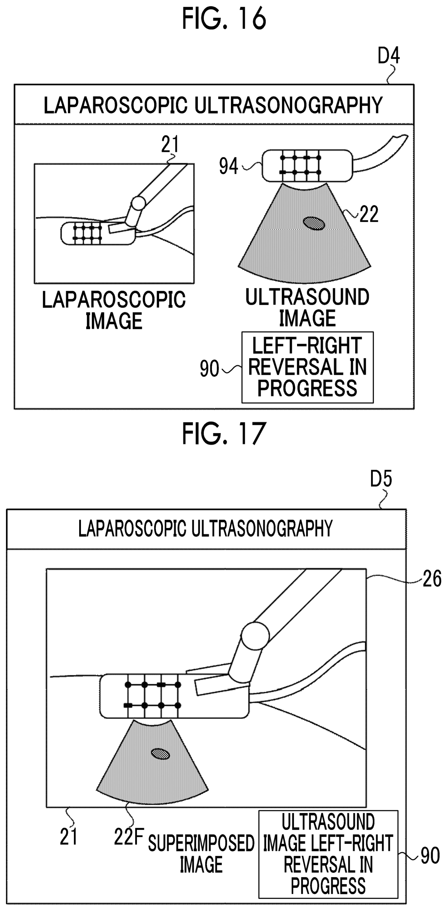

In addition, the first embodiment and the second embodiment may be combined. That is, the display controller 54 may perform control of displaying the information indicating the direction of the ultrasound probe 14 around the ultrasound image 22 while displaying the ultrasound image 22 in a left-right reversed manner in a case where the angle φ is equal to or greater than a predetermined threshold value. FIG. 16 shows an example of a screen D4 displayed on the display 45 by the display controller 54. The screen D4 includes the surgical field image 21, the ultrasound image 22 that is reversed left and right, and the schematic diagram 94 of the ultrasound probe 14. The schematic diagram 94 is reversed left and right to match the ultrasound image 22 that is reversed left and right, unlike the example of FIG. 15. With such a form, it becomes easier to visually grasp the correspondence relationship of the position of the ultrasound probe 14 in the surgical field SF, and the operability of the ultrasound probe 14 via the camera 13B can be improved.

### Third Embodiment

In the present embodiment, as another adjustment method of the display mode of the ultrasound image 22, a form in which the ultrasound image 22 is superimposed and displayed on the surgical field image 21 will be described. In the following description, a part of the description overlapping with the first embodiment or the second embodiment will be omitted.

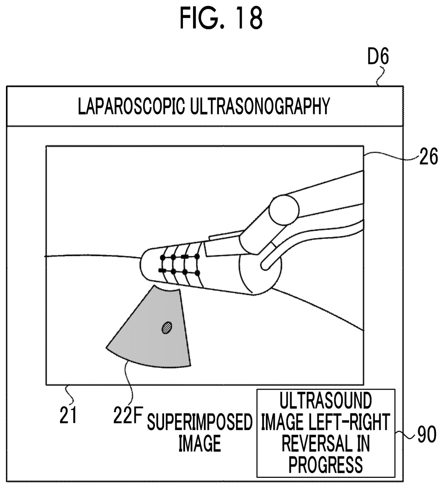

The display controller 54 performs control of displaying a superimposed image 26 obtained by superimposing the ultrasound image 22 on the surgical field image 21, the superimposed image 26 including the ultrasound image 22 whose display mode and display position are adjusted based on the orientation information and the position information of the ultrasound probe 14. FIGS. 17 and 18 show examples of a screen D5 and a screen D6 displayed on the display 45 by the display controller 54.

The screen D5 and the screen D6 include the superimposed image 26 and a notification 90 saying "ultrasound image left-right reversal in progress'". The position and the orientation of the ultrasound probe 14 differ between the screen D5 and the screen D6. The screen D5 corresponds to the state of FIGS. 4 and 5. The screen D6 corresponds to the state of FIGS. 6 and 7.

Specifically, the display controller 54 estimates the position and the orientation of the ultrasound transducer in the surgical field SF based on the orientation information and the position information of the ultrasound probe 14 and the dimension information 59. A linear distance between a reference point of the position indicated by the position information and the ultrasound transducer, and a tilt angle of the ultrasound transducer with respect to the direction of the axis AX indicated by the orientation information are known information defined as the dimension information 59. By using the dimension information 59, the position and the orientation of the ultrasound transducer in the surgical field SF can be estimated from the orientation information and the position information of the ultrasound probe 14.

The display controller 54 estimates the imaging range of the ultrasound image 22 in the surgical field SF based on the estimated position and orientation of the ultrasound transducer. The imaging range of the ultrasound image 22 corresponds to the position and the orientation of the ultrasound transducer. For example, the imaging range of the ultrasound image 22 captured by a convex type ultrasound transducer is a fan-shaped range that spreads radially with the ultrasound transducer as a base point (see FIGS. 4 and 6).

Then, the display controller 54 generates the superimposed image 26 by superimposing the ultrasound image 22 on a region in the surgical field image 21 (Xin-Yin plane) corresponding to the imaging range (Xpb-Ypb plane) of the ultrasound image 22 in the surgical field SF. In the example of FIG. 17, the imaging range (Xpb-Ypb plane) of the ultrasound image 22 in the surgical field SF and the screen (Xin-Yin plane) of the surgical field image 21 in the surgical field SF are parallel to each other. On the other hand, in the example of FIG. 18, the imaging range (Xpb-Ypb plane) of the ultrasound image 22 in the surgical field SF and the screen (Xin-Yin plane) of the surgical field image 21 in the surgical field SF are not parallel to each other. The display controller 54 performs deformation such as projective transformation, affine transformation, movement, rotation, enlargement, and reduction on the ultrasound image 22 in consideration of a difference in coordinate systems between the surgical field image 21 and the ultrasound image 22, and then generates the superimposed image 26 superimposed on the surgical field image 21.

In addition, the display controller 54 adjusts the display mode of the ultrasound image 22 depending on whether or not the angle φ between the direction of the ultrasound probe 14 indicated by the orientation information and the predetermined reference direction satisfies a predetermined criterion. For example, in the examples of FIGS. 17 and 18, the ultrasound image 22 is reversed left and right as in the first embodiment. As in the second embodiment, control may be performed such that the information indicating the direction of the ultrasound probe 14 is displayed around the ultrasound image 22 or is superimposed and displayed on the ultrasound image 22.

With the superimposed image 26, for example, even in a case in which the position and the orientation of the ultrasound probe 14 cannot be visually recognized or are difficult to be visually recognized in the surgical field image 21 due to shield by an organ or the like and a positional relationship with the camera 13B, the position and the orientation can be easily grasped. Therefore, it is easy to adjust the position and the orientation of the ultrasound probe 14 so that a desired region (for example, the tumor 27) is shown in the ultrasound image 22, and it is possible to contribute to the improvement of the operability.

In each of the above-described embodiments, a form has been described in which the orientation information of the ultrasound probe 14 is three-dimensional orientation information, but the present disclosure is not limited to this. The orientation information of the ultrasound probe 14 may be two-dimensional. For example, the derivation unit 52 may derive two-dimensional orientation information of the ultrasound probe 14 based on the surgical field image 21. In this case, the "reference direction" used for calculating the angle φ may be a predetermined direction in the two-dimensional coordinate system (Xin-Yin plane) on the surgical field image 21.

In each of the above-described embodiments, a form has been described in which the position and the orientation of the ultrasound probe 14 in the surgical field SF are derived by using the marker 14M, but the present disclosure is not limited to this. For example, the position and the orientation may be derived by detecting a characteristic shape of the ultrasound probe 14 from the surgical field image 21 through image analysis. In addition, for example, the position and the orientation of the ultrasound probe 14 may be derived by using an AI technology using a machine learning model. As such a machine learning model, for example, a neural network model such as a CNN, which has been trained in advance to receive the surgical field image 21 as an input and output the position and the orientation of the ultrasound probe 14 in the input surgical field image 21, can be applied.

In each of the above-described embodiments, a form has been described in which the state in which the surgical field SF is being scanned with the ultrasound probe 14 is captured by the camera 13B provided in one endoscope 13, but the present disclosure is not limited to this, and the number of the cameras 13B may be plural. For example, a plurality of endoscopes 13 may be inserted into the surgical field SF (at different positions and/or angles), or the camera 13B may be provided on the trocar 17 and a body wall in the body. In this case, the surgical field image 21 used for each processing may be designated by the user or may be automatically switched by the processor 41.

Specifically, the acquisition unit 50 acquires the surgical field image 21 captured by each camera 13B, and, in a case where the user designates which surgical field image 21 to use, the acquisition unit 50 receives the designation. The derivation unit 52 derives the orientation information of the ultrasound probe 14 based on the designated surgical field image 21. The display controller 54 adjusts the display mode of the ultrasound image 22 captured by the ultrasound probe 14 based on the orientation information of the ultrasound probe 14 in the designated surgical field image 21. In a case where the designated surgical field image 21 is changed, each functional unit performs each processing based on the changed surgical field image 21.

In addition, in a case where the processor 41 automatically switches which surgical field image 21 to use, for example, it is considered to select the surgical field image 21 in which the ultrasound probe 14 is easily visible. The acquisition unit 50 acquires the surgical field image 21 captured by each camera 13B. The derivation unit 52 derives the orientation information of the ultrasound probe 14 based on each surgical field image 21. The display controller 54 selects the surgical field image 21 based on the orientation information of each ultrasound probe 14, and adjusts the display mode of the ultrasound image 22 captured by the ultrasound probe 14 based on the orientation information of the ultrasound probe 14 in the selected surgical field image 21. For example, as the angle θ (see FIGS. 4 and 6) representing the direction of the ultrasound image 22 is smaller, it is more difficult to grasp the position and the orientation of the ultrasound probe 14 in the surgical field image 21. Therefore, the display controller 54 may select the surgical field image 21 in which the angle θ representing the direction of the ultrasound image 22 is close to vertical.

In addition, for example, the display controller 54 may select the surgical field image 21 captured by the camera 13B close to the ultrasound probe 14 based on the position information of the ultrasound probe 14. As a result, the ultrasound probe 14 is largely displayed in the surgical field image 21, so that the operability can be improved. In addition, for example, the display controller 54 may combine the above examples to select the surgical field image 21 based on the orientation information and the position information of the ultrasound probe 14. In a case where the position relationship between the ultrasound probe 14 and each camera 13B is changed by moving the ultrasound probe 14 and/or the camera 13B, the display controller 54 may redo the selection of the surgical field image 21.

In each of the above-described embodiments, the body cavity such as the abdominal cavity and the thoracic cavity has been described as the surgical field SF as an example, but the present disclosure is not limited to this. For example, as the surgical field SF, an upper gastrointestinal tract such as the esophagus, a lower gastrointestinal tract such as the intestine, or a tubular organ such as a bronchus may be used.

In each of the above-described embodiments, a form has been described in which the medical support system 10 according to the present disclosure is applied to the robotic surgery, but the present disclosure is not limited to this. For example, the medical support system 10 according to the present disclosure may be applied to an endoscopic surgery. In addition, for example, the medical support system 10 according to the present disclosure may be applied to a remote surgery via a body surface by using a webcam as the camera and using an ultrasound probe that scans the body surface as the ultrasound probe.

In the present embodiment, each processing is executed by any computer. In addition, any computer may execute these processes using a processor as hardware, a program as software, or a combination thereof. In that case, the processor is configured to execute various processes in the present embodiment in cooperation with the program and can function as each unit or each means in the present embodiment. Further, the execution order of the process by the processor is not limited to the order described above and may be changed as appropriate. Any computer may be a general-purpose computer, a computer for a specific use, a workstation, or another system capable of executing each process.

The processor may be configured by one or more pieces of hardware, and the type of hardware is not limited. For example, the processor can be configured with a central processing unit (CPU), a micro processing unit (MPU), a programmable logic device such as a field programmable gate array (FPGA), a dedicated circuit for executing specific processing such as an application specific integrated circuit (ASIC), or hardware such as a graphic processing unit (GPU) or a neural processing unit (NPU). In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each process executed by the processor is not limited to the above order and may be changed as appropriate. The hardware is configured by an electric circuit (circuitry) in which circuit elements such as semiconductor elements are combined.

Further, the program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

In addition, in the above-described embodiment, the medical support program 44 has been described as being stored (installed) in the storage 43 in advance, but the present disclosure is not limited to this. The medical support program 44 may be provided in a form of being recorded on a recording medium, such as a compact disc read only memory (CD-ROM), a digital versatile disc read only memory (DVD-ROM), or a universal serial bus (USB) memory. In addition, the medical support program 44 may be downloaded from an external device via a network.

The technology of the present disclosure extends to any program products. The program product includes a product in any aspect for providing a program. For example, the program product includes a program provided through a network such as the Internet, and a non-transitory computer-readable recording media such as a CD-ROM, a DVD-ROM, and a USB memory in which the program is stored.

In the technology of the present disclosure, the embodiment and the modification examples described above can be combined as appropriate. The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts according to the embodiments of the technology of the present disclosure. As a result, it goes without saying that unnecessary parts may be deleted, new elements may be added, or replacements may be made with respect to the above-described contents and the above-shown contents within a range that does not deviate from the gist of the technology of the present disclosure.

Further, the following Supplementary Notes will be disclosed with regard to the above embodiments.
Supplementary Note 1
   A medical support device comprising:
   a processor,
   in which the processor is configured to
      acquire a surgical field image captured by a camera, the surgical field image showing a state in which a surgical field is being scanned with an ultrasound probe,
      derive orientation information of the ultrasound probe based on the surgical field image, and
      adjust a display mode of an ultrasound image captured by the ultrasound probe based on the orientation information.
Supplementary Note 2
   The medical support device according to Supplementary Note 1,
   in which the processor is configured to
   calculate an angle between a direction of the ultrasound probe indicated by the orientation information and a predetermined reference direction, and
   adjust the display mode depending on whether or not the angle satisfies a predetermined criterion.
Supplementary Note 3
   The medical support device according to Supplementary Note 2,
   in which the processor is configured to perform control of displaying the ultrasound image in a left-right reversed or up-down reversed manner in a case where the angle is equal to or greater than a predetermined threshold value.
Supplementary Note 4
   The medical support device according to Supplementary Note 2 or 3,
   in which the reference direction is a predetermined direction in a three-dimensional coordinate system, and
   the processor is configured to derive three-dimensional orientation information of the ultrasound probe based on the surgical field image.
Supplementary Note 5
   The medical support device according to Supplementary Note 2 or 3,
   in which the reference direction is a predetermined direction in a two-dimensional coordinate system on the surgical field image, and
   the processor is configured to derive two-dimensional orientation information of the ultrasound probe based on the surgical field image.
Supplementary Note 6
   The medical support device according to any one of Supplementary Notes 1 to 5,
   in which the processor is configured to perform control of displaying information indicating a direction of the ultrasound probe around the ultrasound image or displaying and superimposing the information on the ultrasound image, based on the orientation information.
Supplementary Note 7
   The medical support device according to any one of Supplementary Notes 1 to 6,
   in which the processor is configured to
   determine over time whether or not the ultrasound probe is in contact with a target part to be imaged by the ultrasound probe,
   adjust the display mode in a separated state in which the ultrasound probe is separated from the target part, and
   fix the display mode in a contact state in which the ultrasound probe is in contact with the target part.
Supplementary Note 8
   The medical support device according to Supplementary Note 7,
   in which the processor is configured to determine whether the ultrasound probe is in the separated state or in the contact state based on at least one of an electric signal corresponding to a reflected wave of an ultrasound wave transmitted to the target part by the ultrasound probe or the ultrasound image.
Supplementary Note 9
   The medical support device according to any one of Supplementary Notes 1 to 8,
   in which the processor is configured to
   derive the orientation information and position information of the ultrasound probe in the surgical field based on the surgical field image, and
   perform control of displaying a superimposed image obtained by superimposing the ultrasound image on the surgical field image, the superimposed image including the ultrasound image whose display mode and display position are adjusted based on the orientation information and the position information.
Supplementary Note 10
   The medical support device according to any one of Supplementary Notes 1 to 9,
   in which the camera is an endoscope, and
   the ultrasound probe is inserted into a body and images an internal structure of a target part in the body while in contact with the target part.
Supplementary Note 11
   A medical support method executed by a computer, the medical support method comprising:
   acquiring a surgical field image captured by a camera, the surgical field image showing a state in which a surgical field is being scanned with an ultrasound probe;
   deriving orientation information of the ultrasound probe based on the surgical field image; and
   adjusting a display mode of an ultrasound image captured by the ultrasound probe based on the orientation information.
Supplementary Note 12
   A medical support program for causing a computer to execute:
   acquiring a surgical field image captured by a camera, the surgical field image showing a state in which a surgical field is being scanned with an ultrasound probe;
   deriving orientation information of the ultrasound probe based on the surgical field image; and
   adjusting a display mode of an ultrasound image captured by the ultrasound probe based on the orientation information.

## Claims

1. A medical support device comprising a processor, wherein the processor is configured to:
acquire a surgical field image captured by a camera, the surgical field image showing a state in which a surgical field is being scanned with an ultrasound probe;
derive orientation information of the ultrasound probe based on the surgical field image; and
adjust a display mode of an ultrasound image captured by the ultrasound probe based on the orientation information.

2. The medical support device according to claim 1, wherein the processor is configured to:
calculate an angle between a direction of the ultrasound probe indicated by the orientation information and a predetermined reference direction; and
adjust the display mode depending on whether or not the angle satisfies a predetermined criterion.

3. The medical support device according to claim 2, wherein the processor is configured to perform control of displaying the ultrasound image in a left-right reversed or up-down reversed manner in a case where the angle is equal to or greater than a predetermined threshold value.

4. The medical support device according to claim 2 or 3, wherein:
the reference direction is a predetermined direction in a three-dimensional coordinate system, and
the processor is configured to derive three-dimensional orientation information of the ultrasound probe based on the surgical field image.

5. The medical support device according to claim 2 or 3, wherein:
the reference direction is a predetermined direction in a two-dimensional coordinate system on the surgical field image, and
the processor is configured to derive two-dimensional orientation information of the ultrasound probe based on the surgical field image.

6. The medical support device according to any one of claims 1 to 5, wherein the processor is configured to perform control of displaying information indicating a direction of the ultrasound probe around the ultrasound image or displaying and superimposing the information on the ultrasound image, based on the orientation information.

7. The medical support device according to any one of claims 1 to 6, wherein the processor is configured to:
determine over time whether or not the ultrasound probe is in contact with a target part to be imaged by the ultrasound probe;
adjust the display mode in a separated state in which the ultrasound probe is separated from the target part; and
fix the display mode in a contact state in which the ultrasound probe is in contact with the target part.

8. The medical support device according to claim 7, wherein the processor is configured to determine whether the ultrasound probe is in the separated state or in the contact state based on at least one of an electric signal corresponding to a reflected wave of an ultrasound wave transmitted to the target part by the ultrasound probe or the ultrasound image.

9. The medical support device according to any one of claims 1 to 8, wherein the processor is configured to:
derive the orientation information and position information of the ultrasound probe in the surgical field based on the surgical field image; and
perform control of displaying a superimposed image obtained by superimposing the ultrasound image on the surgical field image, the superimposed image including the ultrasound image whose display mode and display position are adjusted based on the orientation information and the position information.

10. The medical support device according to any one of claims 1 to 9, wherein:
the camera is an endoscope, and
the ultrasound probe is inserted into a body and images an internal structure of a target part in the body while in contact with the target part.

11. A medical support method executed by a computer, the medical support method comprising:
acquiring a surgical field image captured by a camera, the surgical field image showing a state in which a surgical field is being scanned with an ultrasound probe;
deriving orientation information of the ultrasound probe based on the surgical field image; and
adjusting a display mode of an ultrasound image captured by the ultrasound probe based on the orientation information.

12. A computer-readable storage medium storing a medical support program for causing a computer to execute:
acquiring a surgical field image captured by a camera, the surgical field image showing a state in which a surgical field is being scanned with an ultrasound probe;
deriving orientation information of the ultrasound probe based on the surgical field image; and
adjusting a display mode of an ultrasound image captured by the ultrasound probe based on the orientation information.
